Europäisches Patentamt

European Patent Office

Office européen des brevets

(19)

(11) Veröffentlichungsnummer : **0 088 871**
**B1**

(12) **EUROPÄISCHE PATENTSCHRIFT**

(45) Veröffentlichungstag der Patentschrift :
28.05.86

(21) Anmeldenummer : **83100811.5**

(22) Anmeldetag : **28.01.83**

(51) Int. Cl.⁴ : **A 61 M 25/00, F 16 K 7/06**

(54) **Katheterventil.**

(30) Priorität : **15.03.82 CH 1593/82**

(43) Veröffentlichungstag der Anmeldung :
**21.09.83 Patentblatt 83/38**

(45) Bekanntmachung des Hinweises auf die Patenterteilung : **28.05.86 Patentblatt 86/22**

(84) Benannte Vertragsstaaten :
**AT BE CH DE FR GB IT LI LU NL SE**

(56) Entgegenhaltungen :
**WO-A-80 /014 54**
**DE-B- 1 077 932**
**FR-A- 2 439 582**
**US-A- 4 056 116**

(73) Patentinhaber : **Stäubli, Hugo**
**Einsiedlerstrasse 240**
**CH-8810 Horgen (CH)**

(72) Erfinder : **Stäubli, Hugo**
**Einsiedlerstrasse 240**
**CH-8810 Horgen (CH)**

(74) Vertreter : **Troesch, Hans Alfred, Dr. Ing. et al**
**Walchestrasse 19**
**CH-8035 Zürich (CH)**

Jouve, 18, rue St-Denis, 75001 Paris, France

## Beschreibung

Die vorliegende Erfindung betrifft ein Katheterventil mit einem zum Verbinden mit dem Katheterschlauch dienenden Ende sowie mit zwei durch einen gummielastischen Ventilschlauch verbundenen Elementen, welche bei offenem Ventil einen Durchgang festlegen und bei geschlossenem Ventil diesen Durchgang im Ventilschlauch unterbrechen.

Es ist bisher üblich, den Katheterschlauch mit Hilfe eines konischen Verschlusszapfens zu verschliessen. Dieser, seit Jahrzehnten verwendete Verschlusszapfen, muss bei jedem Urinieren entfernt und anschliessend wieder in das Schlauchende eingeführt werden. Abgesehen davon, dass das Einsetzen des Zapfens wegen der relativ kleinen Schlauchmündung und dem möglicherweise schlecht zugänglichen Ort mühsam ist, gilt dies insbesondere für ältere Patienten, welche oftmals umsonst mit zittrigen Händen versuchen, den Zapfen kunstgerecht zu plazieren.

Es ist ferner eine Harnenthnahmevorrichtung bekannt (WO-A-80/01454), die ein Drainieren der Blase ermöglicht und einen Katheter besitzt, der mit der Blase verbindbar ist und mit einem Auffangbeutel in Verbindung steht. Diese Vorrichtung besitzt ein Ventil, das zur Steuerung der Verbindung zwischen dem Katheter und dem Auffangbeutel dient und einen mit dem Katheter verbundenen Einlass, einen mit dem Auffangbeutel verbundenen Auslass und eine Membran aufweist. Diese steuert die Verbindung zwischen dem Ein- und dem Auslass. Diese Membran ist so angeordnet, dass sie den Einlass mit einer elastischen Kraft geschlossen hält und ihn öffnet, wenn der Druck in dem Einlass einen vorherbestimmten Wert erreicht. Dabei ist die Membran am Einlass so angeordnet, dass ein in dem Einlass vorhandener Druck, der niedriger ist als der Oeffnungsdruck des Ventils, geeignet ist, die Membran in der Offenstellung zu halten, bis die genannte elastische Kraft stärker ist als die Kraft, die durch den in dem Einlass herrschenden Druckmitteldruck auf die Membran ausgeübt wird.

Die Vorrichtung ist strömungsmässig kompliziert, so dass sich, insbesondere an den Umlenkstellen, Ablagerungen bilden, die die Vorrichtung undicht machen.

Die vorliegende Erfindung bezweckt die Schaffung eines Katheter-Endverschlusses in Form eines Ventils, welches beim normalen Urinieren am Schlauch bleibt und nur zum Spülen vom Schlauchende getrennt werden muss.

Ein derartiges Katheterventil soll auch die Infektionsgefahr vermindern und die Sauberkeit allgemein vergrössern, da das ungewollte Abfliessen des Urins verhütet wird.

Ein derartiges, den vorgenannten Anforderungen entsprechendes Katheterventil zeichnet sich erfindungsgemäss dadurch aus, dass an dem als Festteil mit einer Durchflussbohrung ausgebildeten einen Element das eine Ende des Ventilschlauches befestigt ist und über dem Festteil und dem Schlauch eine mittels einer Feder belastete, mit dem andern Schlauchende verbundene Dreh-, Schiebe- oder Dreh/Schiebehülse angeordnet ist, durch deren Bewegen entgegen der Federkraft der in unbetätigter Ruhelage durch ein- oder mehrfache Knickung, Faltung oder Torsion geschlossene Durchgang in die Oeffnungslage gebracht wird.

Ein Ausführungsbeispiel des Erfindungsgegenstandes wird anschliessend anhand einer Zeichnung erläutert.

Es zeigen :

·Figur 1 ein in seine Einzelteile zerlegtes Katheterventil in perspektivischer Darstellung,

Figur 2 das Katheterventil gemäss Fig. 1 im Längsschnitt, in offenem Zustand,

Figur 3 das Ventil gemäss Fig. 2 in geschlossenem Zustand,

Figur 4 eine Ansicht auf die Schiebehülse von hinten,

Figur 5 einen Längsschnitt durch den Knickschlauch.

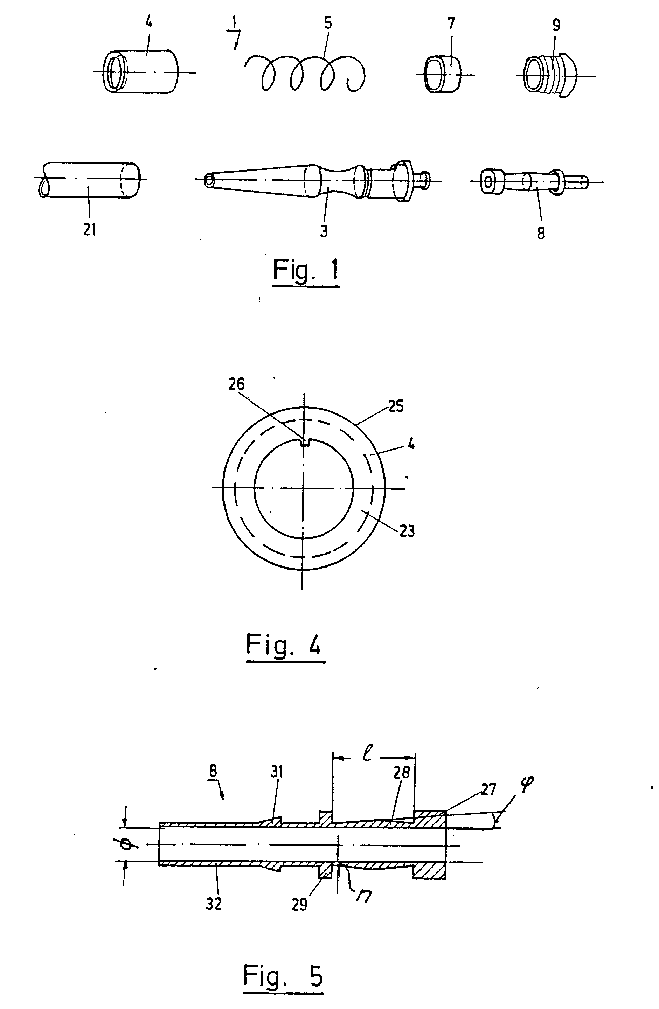

Wie Fig. 1 zeigt, setzt sich das Katheterventil 1 aus sechs Teilen zusammen. Es sind dies der Ventilnippel 3, eine Schiebehülse 4, eine Schraubenfeder 5 aus rostfreiem Stahl, ein Distanzring 7, ein Knickschlauch 8 sowie eine Verschlusskappe 9. Ausser den Teilen 5 und 8 bestehen die übrigen Teile vorzugsweise aus Kunststoff.

Das Katheterventil 1 wird zusammengesetzt, indem die Feder 5 über einen konischen Zapfen 12 des Nippels 3 geschoben wird, gefolgt von der Schiebehülse 4.

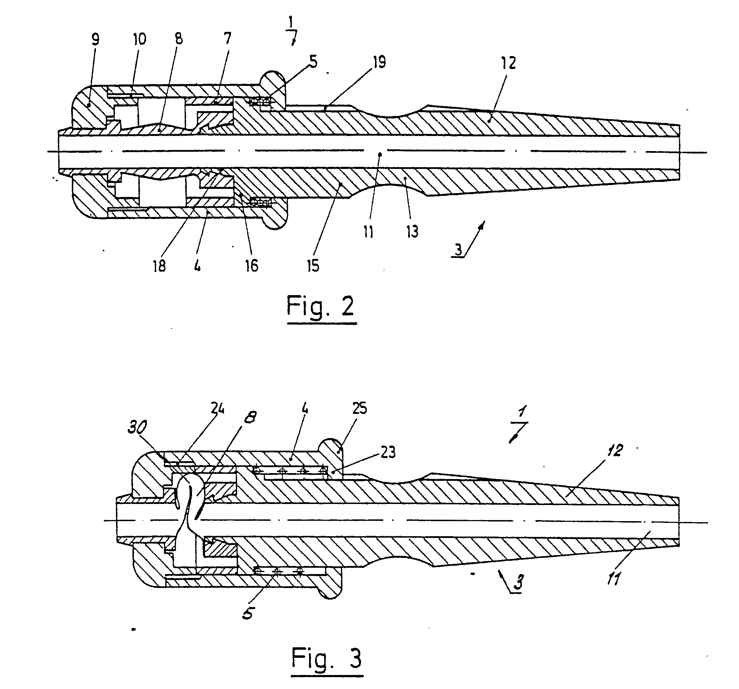

Der Knickschlauch 8 wird auf einen Fortsatz 18 des Ventilnippels 3 aufgeschoben und anschliessend der Distanzring 7 über den Knickschlauch 8 gebracht. Abschliessend wird unter Zusammenpressen der Schraubenfeder 5 die Verschlusskappe 9 mittels ihres Aussengewindes 10 mit der Schiebehülse 4 bzw. deren Innengewinde 24 verschraubt. Nun kann die Schiebehülse 4 losgelassen werden, worauf die Schraubenfeder 5 die Hülse 4 nach hinten presst und dabei den Knickschlauch 8 in die in Fig. 3 dreifach geknickte Lage bringt. Durch nach Vorneschieben der Schiebehülse 4 wird der Knickschlauch 8, d. h. die Knickungen 30 gestreckt, der Durchgang ist frei und das Ventil offen, wie dies Fig. 2 zeigt.

Der Distanzring 7 verhindert im übrigen ein übermässiges Zusammendrücken des Knickschlauches, was zweifellos dessen Lebensdauer verlängert.

Der Ventilnippel 3, der Festteil des Ventils 1, weist eine durchgehende Bohrung 11 auf. Sein hinteres Ende ist als konischer Zapfen 12 ausgebildet, der dazu vorgesehen ist, in das entsprechende Schlauchende 21 eines Katheterschlauches eingeführt zu werden. Dem konischen Zapfen 12 folgt ein Abstützteil 13, welcher beim nach Vorneschieben der Schiebehülse 4 dem betätigenden Daumen als Gegenlager dient. Dem Abstützteil 13 folgt ein Feder-Führungszylinder 15 zum Führen der Schraubenfeder 5. An dessen Ende befindet sich ein Führungsteller 16 zur Führung der Schiebehülse 4. Er dient auch als Federteller für das feste Ende der Schraubenfeder 5. Dem Teller 16 folgt ein Fortsatz 18, welcher der Aufnahme des einen Endes des Knickschlauches 8 dient. Der Ventilnippel 3 ist ferner mit einer Nut 19 versehen, in welche ein Führungsnocken 26 der Schiebehülse 4 gleitet, zum Zweck, ein Drehen der Schiebehülse 4 und damit eine schwierigere Betätigung beim Oeffnen des Ventils zu verhüten.

Die Schiebehülse 4 weist einen Kragen 23 auf, welcher dem verschiebbaren Ende der Schraubenfeder 5 als Federteller dient. Ein Griffwulst 25 erlaubt bein besseres Abstützen des Daumens beim nach Voreschieben der Schiebehülse 4 zwecks Oeffnen des Ventils.

Wie Fig. 5 zeigt, ist der Aufbau des Knickschlauches 8 im Hinblick auf seine Verschlussfunktion recht kompliziert beschaffen. Er weist einen Haltewulst 27 auf, der über den Fortsatz 18 des Ventilnippels 3 gestülpt wird. Ihm folgt der eigentliche Knickteil 28, der bei gleichbleibendem Durchgang erst konisch zunimmt, um von der Mitte ab und entsprechend bis zu eionem Auflagerwulst 29 hin abzunehmen. Nach einem weiteren zylindrischen Teil folgt ein Haltekragen 23, der sich aussen auf der Verschlusskappe 4 abstützt.

Der Knickschlauch 8 endet mit einem Schlauchmündungsteil 32, welcher über die Verschlusskappe 9 vorsteht. Es ist auch möglich, wie die Fig. 2 und 3 zeigen, den Schlauchmündungsteil 32 wegzulassen. Dies weist den Vorteil der geringeren Sperrigkeit beim Vorsorgen des Ventils in der Kleidung auf, bringt aber den Nachteil des wahrscheinlicheren Nässens der übrigen Ventilteile durch Urin.

Der kritische Teil dieses, nach der Bauart eines Quetschventils hergestellten Ventils ist zweifellos der Knickschlauch 8, welcher in Einzelheiten in Fig. 5 ersichtlich ist. Die Konizität φ des doppelkonusförmigen eigentlichen Knickteiles 28 beträgt ca. 5°, d. h. der gesamte Oeffnungswinkel des Konus ist 10°. Bei einem Durchgang φ von 4 mm und einer minimalen Wandstärke η von 0,5 mm an den Uebergangsstellen des Knickteils 28 in den Haltewulst 27 und den Auflagerwulst 29 beträgt die Knicklänge l = 10 mm. Dabei ist zu beachten, dass beim nach Voreschieben der Schiebehülse 4, d. h. beim Oeffnen des Ventils 1, der Schlauch sich vorzugsweise aus eigenen Kräften aus der dreifach geknickten Lage gemäss Fig. 3 in die Strecklage gemäss Fig. 2 bewegen kann. Dabei wird allerdings diese Bewegung selbst bei erlahmtem Schlauch 8 zwangsläufig durch den Haltekragen 31, an dem die Hülse 4 beim nach Voreschieben den Knickschlauch 8 mitnimmt, sichergestellt.

Bei dieser speziellen Formgebung des Knickschlauches 8 wird eine dreifache Knickung und damit ein ganz sicheres Abschliessen des Schlauches bzw. Schliessen des Ventils 1 erreicht.

Das Ventil 1 wird entweder mit beiden Händen oder einhändig mit dem Daumen betätigt. Es muss so lange nach vorne geschoben und gehalten werden, bis aller Urin abgeflossen ist.

Da es grundsätzlich möglich ist, einen Schlauch nicht nur durch ein- oder mehrfaches Knicken zu verschliessen, sondern durch eine Drehbewegung oder eine kombinierte Dreh-Schiebebewegung, kann grundsätzlich die beschriebene Schiebehülse auch als achsial feste Drehhülse oder als sog. Drehschieber mit einer kombinierten Dreh- und Längsbewegung ausgebildet werden. In diesem Falle muss der Schlauch mit dem hier als Hülse ausgebildeten Element — es könnte auch eine von aussen mittels eines Stiftes bewegbare Innenhülse vorgesehen werden — verbunden werden, damit dem Schlauch die nötige Dreh- und/oder Schiebebewegung in achsialer Richtung übermittelt werden kann. Wird eine reine Torsion vorgesehen, so kann anstelle einer Torsionsfeder auch ein Torsionsstab vorgesehen werden.

Dieses Ventil weist neben den angegebenen Vorteilen weiterhin den Vorteil auf, dass es nicht verlorengehen kann, wie die bisherigen Zapfen, und dass es ganz allgemein ein angenehmes Hilfsmittel für Patient und Pflegepersonal bei Dauerkatheter ist.

Da das Ventil keine scharfen Kanten und Ecken besitzt und keine harten vorstehenden Teile, ist die Gefahr des Hängenbleibens an Kleidungsstücken ausserordentlich gering.

Mittels eines Sicherungszäpfchens, einsteckbar im Festteil 3 am hinteren Rand der Hülse 4 bei offenem Ventil (Fig. 2) wird der Schlauch 8 beim Lagern in gestreckter Lage gehalten, was Knickschäden verhütet.

**Patentansprüche**

1. Katheterventil mit einem zum Verbinden mit dem Katheterschlauch (21) dienenden Ende sowie mit zwei durch einen gummielastischen Ventilschlauch (8) verbundenen Elementen, welche bei offenem Ventil einen Durchgang festlegen und bei geschlossenem Ventil diesen Durchgang im Ventilschlauch unterbrechen, dadurch gekennzeichnet, dass an dem als Festteil (3) mit einer Durchflussbohrung (11) ausgebildeten einen Element das eine Ende des Ventilschlauches (8) befestigt ist und über dem Festteil (3) und dem Schlauch (8) eine mittels einer Feder (5) belastete, mit dem anderen Schlauchende verbundene Dreh-, Schiebe- oder Dreh/Schiebehülse (4) angeordnet ist, durch deren Bewegen entgegen der Federkraft der in unbetätigter Ruhelage durch ein- oder mehrfache Knickung (30), Faltung oder Torsion geschlossene Durchgang in die Oeffnungslage gebracht wird.

2. Katheterventil nach Anspruch 1, dadurch gekennzeichnet, dass das Ventil (1) eine Dreh-(19) bzw. Schiebesicherung (26) für die das Ventil (1) betätigende Hülse (4) aufweist.

3. Katheterventil nach Anspruch 1 oder 2, dadurch gekennzeichnet, dass der Schlauch (8) einen speziellen Knick- oder Torsionsteil (28) aufweist, welcher vorzugsweise an seinen Enden eine geringere Wandstärke aufweist als in der Mitte, wobei z. B. die Bohrung (11) zylindrisch ist und die Mantelfläche, von beiden Enden ausgehend, sich konisch erweitert.

4. Katheterventil nach einem der Ansprüche 1 bis 3, dadurch gekennzeichnet, dass im Festteil (3) ein Abstützteil (13) für die das Ventil (1) öffnende Hand vorgesehen ist.

## Claims

1. A catheter valve with an end used for connecting to the flexible catheter tube (21) together with two elements joined by an elastic valve tube (8) and which with the valve open establish a passage and with the valve closed interrupt said passage in the valve tube, characterised in that one end of the valve tube (8) is affixed to one element formed as a fixed portion (3) with a flow bore, and over the fixed portion (3) and the tube (8) is disposed a rotary and/or slidable sleeve (4) loaded by a spring (5) and joined to the other end of the valve tube, whereby on movement of the sleeve against the force of the spring, the passage which in the unactuated rest position is closed by single or multiple kinking (30), folding or torsion is taken into the open position.

2. Catheter valve as in Claim 1, characterised in that the valve (1) includes means (19) for preventing rotational movement or means (26) for preventing translatory movement of the sleeve (4) which actuates the valve (1).

3. Catheter valve as in Claim 1 or 2, characterised in that the valve tube (8) has a special kinking or torsion portion (28), preferably with lesser wall thickness at its ends than in the centre, wherein for example the bore (11) is cylindrical and the outer surface widens conically from both ends.

4. Catheter valve as in any one of Claims 1-3, characterised in that the fixed portion (3) includes a section (13) to be engaged by the hand opening the valve (1).

## Revendications

1. Valve pour cathéter, comportant une extrémité destinée à être raccordée au tuyau (21) du cathéter, ainsi que deux éléments reliés par un tuyau souple de valve (8) présentant l'élasticité du caoutchouc, ces éléments établissant un passage à travers le tuyau de valve dans la position d'ouverture de la valve et supprimant ce passage dans la position de fermeture de la valve, caractérisée en ce qu'une extrémité du tuyau de valve (8) est fixée sur l'un des éléments réalisé sous la forme d'une pièce fixe (3) munie d'un perçage (11) pour l'écoulement et en ce que, autour de la pièce fixe (3) et du tuyau (8), se trouve disposé un manchon rotatif ou coulissant ou à la fois rotatif et coulissant (4), chargé par un ressort (5) et relié à la seconde extrémité du tuyau, le passage, qui dans l'état non actionné de repos est obturé par un écrasement, un pliage ou une torsion simple ou multiple (30), étant amené dans la position ouverte sous l'effet du déplacement du manchon à l'encontre de la force du ressort.

2. Valve pour cathéter selon la revendication 1, caractérisée en ce que la valve (1) comporte un moyen de blocage en rotation (19) ou de blocage en translation (26), pour le manchon (4) actionnant la valve (1).

3. Valve pour cathéter selon la revendication 1 ou 2, caractérisée en ce que le tuyau (8) possède un tronçon particulier pouvant être plié ou tordu (28) et présentant de préférence une épaisseur de paroi plus faible au niveau de ses extrémités que dans sa partie médiane, le perçage (11) par exemple étant cylindrique et la surface d'enveloppe s'élargissant sous une forme conique à partir des deux extrémités.

4. Valve pour cathéter selon l'une des revendications 1 à 3, caractérisée en ce qu'il est prévu, dans la pièce fixe (3), une zone d'appui (13) pour la main ouvrant la valve (1).

**Fig. 1**

**Fig. 4**

**Fig. 5**

Fig. 2

Fig. 3